# EUROPEAN PATENT SPECIFICATION

(11) **EP 4 632 342 B1**
(45) Date of publication and mention of the grant of the patent: **19.08.2026**
(21) Application number: 23900390.8
(22) Date of filing: 10.11.2023
(51) Int. Cl.: G01J 5/48, A61B 5/01

(54) **HUMAN-BODY SURFACE TEMPERATURE CALCULATION SYSTEM, HUMAN-BODY SURFACE TEMPERATURE CALCULATION METHOD, AND PROGRAM**
SYSTEM ZUR BERECHNUNG DER OBERFLÄCHENTEMPERATUR DES MENSCHLICHEN KÖRPERS, VERFAHREN ZUR BERECHNUNG DER OBERFLÄCHENTEMPERATUR DES MENSCHLICHEN KÖRPERS UND PROGRAMM
SYSTÈME DE CALCUL DE TEMPÉRATURE DE SURFACE DE CORPS HUMAIN, PROCÉDÉ DE CALCUL DE TEMPÉRATURE DE SURFACE DE CORPS HUMAIN, ET PROGRAMME

(30) Priority: 09.12.2022 JP 2022197394
(43) Date of publication of application: 15.10.2025
(73) Proprietor: Panasonic Intellectual Property Management Co., Ltd., Kadoma-shi, Osaka 571-0057 (JP)
(72) Inventor: ITOH, Kazuo, Kadoma-shi 571-0057 Osaka (JP); KOGA, Tatsuo, Kadoma-shi 571-0057 Osaka (JP)
(74) Representative: Appelt, Christian W.
(86) International application number: PCT/JP2023/040505
(87) International publication number: WO 2024/122269

(56) References cited:
- WO-A1-2023/188797
- CN-A- 111 189 544
- JP-A- 2013 024 761
- JP-A- 2016 080 620
- JP-A- 2022 181 356
- JP-A- H10 197 348
- US-A1- 2019 182 439
- US-A1- 2022 269 894

## Description

### [Technical Field]

The present invention relates to a human body surface temperature calculation system, a human body surface temperature calculation method, and a program.

### [Background Art]

A method for estimating an amount of clothing of a clothed portion based on a temperature of the clothed portion of a subject that is visible in a thermal image indicating a temperature distribution of a target space and clothing information on clothing, and for estimating an apparent temperature of a user (Patent Literature (PTL) 1, for example) is known.

### [Citation List]

### [Patent Literature]

[PTL 1] WO 2020/084777

### [Summary of Invention]

### [Technical Problem]

The present invention provides a human body surface temperature calculation system, a human body surface temperature calculation method, and a program that can accurately calculate a surface temperature of a person present in a target space.

### [Solution to Problem]

A human body surface temperature calculation system according to one aspect of the present invention includes: a thermal image generator that generates a thermal image of a target space based on temperature distribution data indicating a temperature distribution of the target space obtained by an infrared sensor; an identifier that identifies a person detection area using a person detection artificial intelligence (AI), the person detection area being a rectangular area that includes a person visible in the thermal image; a first calculator that extracts a first temperature value group corresponding to the person detection area from the temperature distribution data, and calculates, based on the first temperature value group extracted, a temperature of a background area and an average temperature of the person detection area, the background area being an area that is included in the person detection area and in which a background excluding the person is visible; and a second calculator that extracts a second temperature value group from the first temperature value group based on a rule determined by a magnitude relationship between the temperature of the background area calculated and the average temperature of the person detection area calculated, and calculates a surface temperature of the person based on the second temperature value group extracted.

A human body surface temperature calculation method according to one aspect of the present invention is a human body surface temperature calculation method that is executed by a computer, and the human body surface temperature calculation method includes: generating a thermal image of a target space based on temperature distribution data indicating a temperature distribution of the target space obtained by an infrared sensor; identifying a person detection area using a person detection artificial intelligence (AI), the person detection area being a rectangular area that includes a person visible in the thermal image; extracting a first temperature value group corresponding to the person detection area from the temperature distribution data, and calculating, based on the first temperature value group extracted, a temperature of a background area and an average temperature of the person detection area, the background area being an area that is included in the person detection area and in which a background excluding the person is visible; and extracting a second temperature value group from the first temperature value group based on a rule determined by a magnitude relationship between the temperature of the background area calculated and the average temperature of the person detection area calculated, and calculating a surface temperature of the person based on the second temperature value group extracted.

A program according to one aspect of the present invention is a program for causing the computer to execute the above-mentioned human body surface temperature calculation method.

### [Advantageous Effects of Invention]

The human body surface temperature calculation system, the human body surface temperature calculation method, and the program according to the present invention can accurately calculate a surface temperature of a person present in a target space.

### [Brief Description of Drawings]

[FIG. 1]

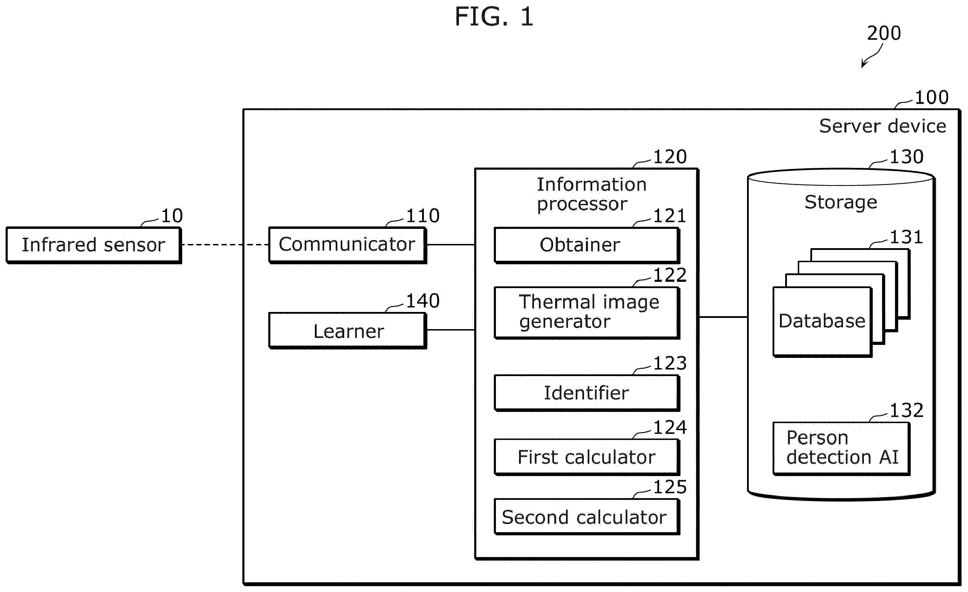

   FIG. 1 is a block diagram of a functional configuration of a human body surface temperature calculation system according to an embodiment.
[FIG. 2]

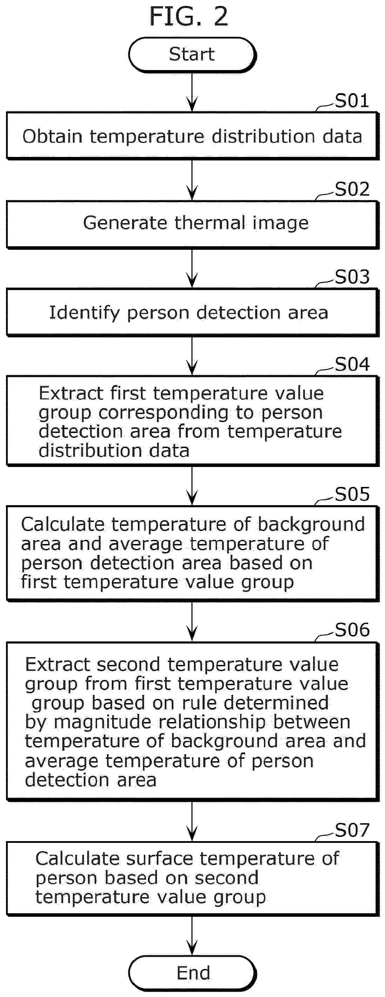

   FIG. 2 is a flowchart illustrating an operation example of the human body surface temperature calculation system according to the embodiment.
[FIG. 3]

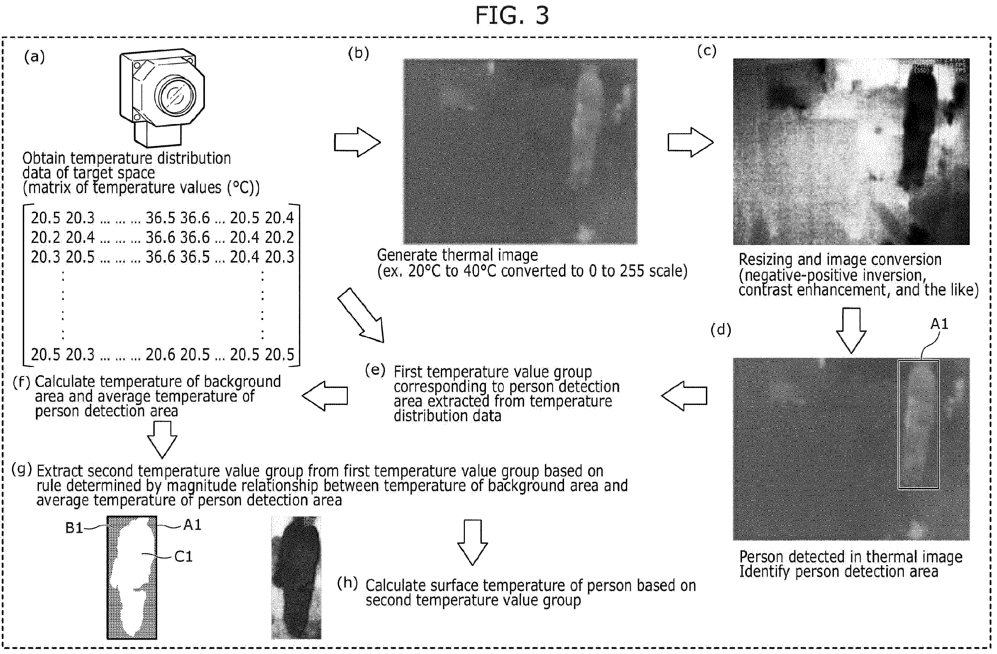

   FIG. 3 is a diagram schematically illustrating the flowchart illustrated in FIG. 2.
[FIG. 4]

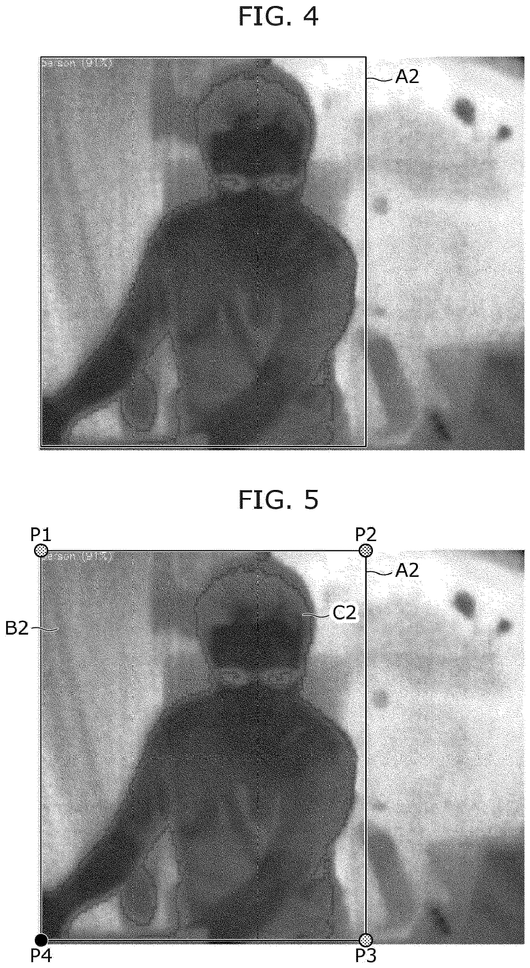

   FIG. 4 is a diagram illustrating an example of a thermal image.
[FIG. 5]
   FIG. 5 is a diagram for describing a first example of a process for a first calculation step.
[FIG. 6]

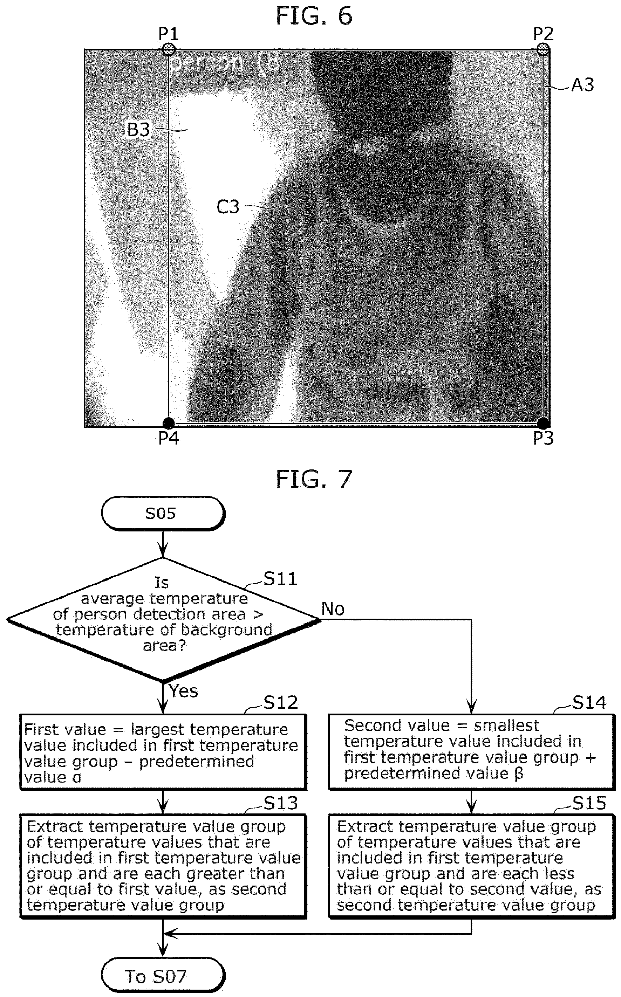

   FIG. 6 is a diagram for describing a second example of a process for the first calculation step.
[FIG. 7]
   FIG. 7 is a flowchart illustrating an example of a detailed flow of step S06 in FIG. 2.
[FIG. 8]

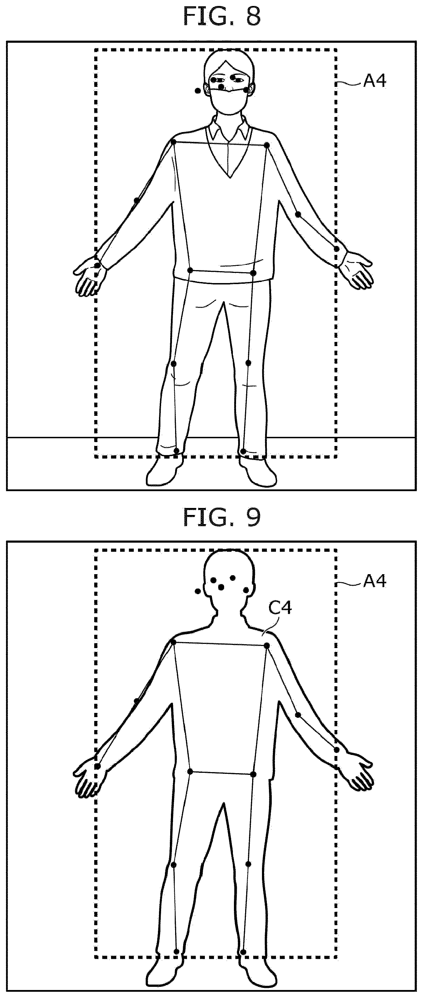

   FIG. 8 is a diagram illustrating a person detection area detected in a thermal image by a posture estimating AI.
[FIG. 9]
   FIG. 9 is a diagram illustrating an example of an area defined by lines connecting different nodes in a skeletal frame model of a body of a person.

### [Description of Embodiments]

Hereinafter, exemplary embodiments will be described with reference to the drawings. It should be noted that the embodiments described below merely illustrate general or specific examples of the present disclosure. The numerical values, shapes, materials, elements, the arrangement and connection of the elements, steps, the order of the steps, etc., described in the following embodiment are mere examples, and are therefore not intended to limit the present disclosure. Accordingly, among elements in the following embodiments, those not appearing in any of the independent claims will be described as optional elements.

It should be noted that the respective figures are schematic diagrams and are not necessarily precise illustrations. Furthermore, in the respective figures, elements that are substantially the same are given the same reference signs, and redundant descriptions may be omitted or simplified.

### (Embodiment)

A human body surface temperature calculation system according to the present embodiment generates a thermal image based on temperature distribution data of a target space, identifies a person detection area that includes a person visible in the thermal image using a person detection artificial intelligence (AI), extracts a second temperature value group corresponding to an area in which the person is shown from a first temperature value group corresponding to the person detection area based on a predetermined rule, and calculates a surface temperature of the person based on the second temperature value group.

The target space is, for example, a target space in which air conditioning is controlled, but such control is not limited to air conditioning, and the target space may be a target space in which lighting or aromas, or the like, are controlled. The space is, for example, a space in a building, but this example is not limiting, and may be a mobile body, such as an automobile, a train, an airplane, a bus, a marine vessel, or the like. The building is, for example, a residence, an office, an educational facility, an accommodation facility, a leisure facility, a commercial facility, a public facility, a medical facility, a nursing facility, or the like.

The predetermined rule is a rule that is determined by a magnitude relationship between a temperature of a background area included in the person detection area, in which a background excluding the person is visible, and an average temperature of the person detection area. The rule will be described later in detail.

Hereinafter, the human body surface temperature calculation system according to the present embodiment will be specifically described with reference to the drawings.

### [1. Configuration]

First, a configuration of the human body surface temperature calculation system according to the embodiment will be described. FIG. 1 is a block diagram of a functional configuration of human body surface temperature calculation system 200 according to an embodiment.

In the example in FIG. 1, although human body surface temperature calculation system 200 includes infrared sensor 10 and server device 100, it is sufficient if server device 100 is included, and infrared sensor 10 need not be included. Furthermore, in the example in FIG. 1, although human body surface temperature calculation system 200 includes one infrared sensor 10, a plurality of infrared sensors 10 may be included.

### [Infrared Sensor]

Infrared sensor 10 is, for example, provided in a ceiling or a wall, or the like, of the target space, and obtains temperature distribution data that indicates a temperature distribution of the target space viewed from above. It should be noted that infrared sensor 10 may generate a thermal image based on the temperature distribution data obtained. Infrared sensor 10 may, for example, be an infrared light array sensor (thermal image sensor) that includes an M × L (where M and L are integers greater than or equal to 2) array of infrared light detection elements. In other words, the temperature distribution data obtained by infrared sensor 10 is an M row × L column matrix of temperature values, and the thermal image generated based on the temperature distribution data has M × L pixels. The thermal image indicates the temperature distribution in a sensing range of infrared sensor 10 at a resolution of M × L.

For example, infrared sensor 10 may be detachably connected to a power supply terminal included in a lighting device provided in a ceiling of the target space. In this case, infrared sensor 10 operates by receiving power supplied by the lighting device. The power supply terminal is, for example, a universal serial bus (USB). Furthermore, infrared sensor 10 may be directly fixed to a ceiling or a wall of the target space without being connected via the lighting device. Furthermore, by fixing infrared sensor 10 to a wall or the like, infrared sensor 10 may obtain temperature distribution data that indicates a temperature distribution viewed from the side.

It should be noted that the thermal image may be a video image or may be a still image.

### [Server Device]

Server device 100 generates a thermal image of the target space based on temperature distribution data of the target space obtained by infrared sensor 10, identifies a person detection area that includes a person visible in the thermal image using person detection AI 132, and extracts a first temperature value group corresponding to the person detection area from the temperature distribution data. Then, server device 100 extracts a second temperature value group corresponding to an area included in the person detection area, in which the person is shown (also referred to as a "person area") from the first temperature value group based on a predetermined rule, and calculates a surface temperature of the person based on the second temperature value group. Server device 100 is an edge computer provided in a building in which the target space is included, but may be a cloud computer provided outside of the building. Server device 100 includes communicator 110, information processor 120, storage 130, and learner 140, for example

Communicator 110 is a communication module (or a communication circuit) for server device 100 communicating with infrared sensor 10. Communicator 110 receives temperature distribution data of the target space from infrared sensor 10, for example. Communication performed by communicator 110 may be wireless communication, and may be wired communication. The communication standard used for communication is not particularly limited. For example, such communication may be in a format in which analog output from infrared sensor 10 is input to an A/D input port of a microcomputer, and may be in a format in which an infrared image (a so-called, thermal image) is obtained as input from an RGB camera from infrared sensor 10 (an infrared camera, for example) via a USB cable.

Information processor 120 obtains the temperature distribution data that is received from communicator 110, generates a thermal image based on the temperature distribution data obtained, extracts a person detection area from the thermal image generated, and performs information processing for calculating a surface temperature of a person based on a second temperature value group corresponding to a person area in which a person is shown from a first temperature value group corresponding to the person detection area extracted. Information processor 120 is specifically implemented as a program and a processor or a microcomputer that executes the program.

Information processor 120 specifically includes obtainer 121, thermal image generator 122, identifier 123, first calculator 124, and second calculator 125. Obtainer 121 obtains the temperature distribution data of the target space received by communicator 110. Thermal image generator 122 generates a thermal image of the target area based on the temperature distribution data obtained by obtainer 121. Identifier 123 identifies a person detection area that includes a person visible in the thermal image using person detection AI 132. First calculator 124 extracts a first temperature value group corresponding to the person detection area from the temperature distribution data, and calculates, based on the first temperature value group extracted, a temperature of a background area included in the person detection area, in which a background excluding the person is visible, and an average temperature of the person detection area. Second calculator 125 extracts a second temperature value group from the first temperature value group based on a rule (a so-called predetermined rule) determined by a magnitude relationship between the temperature of the background area and the average temperature of the person detection area calculated by first calculator 124, and calculates a surface temperature of the person based on the second temperature value group extracted.

The functionality of obtainer 121, thermal image generator 122, identifier 123, first calculator 124, and second calculator 125 is implemented, for example, by a processor or a microcomputer included in information processor 120 or the like executing a computer program stored in the microcomputer or storage 130. The functionality of obtainer 121, thermal image generator 122, identifier 123, first calculator 124, and second calculator 125 will be described later in detail.

Storage 130 is a storage device in which the temperature distribution data received by communicator 110, a computer program executed by information processor 120, and the like are stored. Storage 130 also stores database 131 and person detection AI 132, and the like. Storage 130 may also store training data (not illustrated in the figures) used to train person detection AI 132. Storage 130 is specifically implemented, for example, as a semiconductor memory or a hard disk drive (HDD), or the like.

Database 131 stores temperature distribution data, a first temperature value group corresponding to a person detection area, and a second temperature value group corresponding to a person area, and the like, for example. Furthermore, database 131 may store information items on seasons, outside temperatures, room temperatures, and predetermined value α and predetermined value β in association with each other.

Stated differently, person detection AI 132 is a person detection AI model, and is an AI that detects a person visible in the thermal image that is input. For example, person detection AI 132 detects a person visible in the thermal image, by receiving the thermal image as input, and outputs information on the position and size, and the like, within the thermal image of the person detection area (the so-called "bounding box") that indicates a rectangular area that includes the person detected. For example, person detection AI 132 may be an object detection AI (or in other words, an object detection AI model), and may be a posture estimation AI (or in other words, a posture estimation AI model). The thermal image input to person detection AI 132 is a thermal image generated by thermal image generator 122 based on a temperature distribution of the target space, but may be a thermal image obtained by resizing and/or performing image conversion of the thermal image.

The object detection AI is an AI that detects an object visible in the image that is input, and categorizes (or in other words, classifies) the object detected. For example, the object detection AI detects an object visible in the image, by receiving the image as input, and outputs information on the position and size, and the like, within the image of the object detection area (the so-called "bounding box") that indicates a rectangular area that includes the object detected, and categorization information (also referred to as categories or classes) that categorizes the object detected. The object detection AI is, for example, a Region-based Convolutional Neural Network (R-CNN), You Only Look Once (YOLO), or Single Shot MultiBox Detector (SSD). In particular, the object detection AI may be an SSD.

Furthermore, the posture estimation AI is an AI that estimates a posture of the person visible in the image that is input. For example, the posture estimation AI may detect the person visible in the image, by receiving the image as input, to detect feature points, such as the joints, the eyes, the nose, and the like, of the person detected, and estimate a skeletal frame (or in other words, the posture) that includes line segments connecting different feature points detected, or may extract all feature points, by receiving the image as input, to estimate, for all of the feature points extracted, a skeletal frame (posture) that includes line segments connecting different feature points that have been matched for each person. For example, by receiving the image as input, the posture estimation AI outputs data to which coordinates of feature points of the person visible in the image and skeletal frame information, and the like, that includes connection information between the different feature points are added. The posture estimation AI is, for example, OpenPose, PoseNet, PifPaf, HigherHRNet, a Cascaded Pyramid Network (CPN), or SimpleBaseline. In particular, the posture estimation AI may be PoseNet.

It should be noted that the above-mentioned models are merely examples, and the models are not limited by these examples. Furthermore, the image input to the above-mentioned object detection AI and posture estimation AI may be a thermal image, and may be a thermal image obtained by resizing and/or performing image conversion on the thermal image.

For example, learner 140 performs machine learning using training data. Training data is, for example, a data set that includes pairs of thermal images received as input data and person detection areas transmitted as output data. Furthermore, the training data that is input data may include a thermal image obtained by resizing and/or performing image conversion on the thermal image. The training data is not limited to the above-mentioned examples, and it is sufficient if pairs of data sets are prepared as needed in accordance with the AI model being used. Learner 140 receives a thermal image indicating the temperature distribution of the target space as input, by machine learning, for example, and generates person detection AI 132 (a so-called person detection AI model) that outputs a person detection area that includes a person visible in the thermal image. Learner 140 updates person detection AI 132 by storing the pre-trained person detection AI 132 in storage 130. Learner 140 is implemented, for example, by a processor executing a program stored in storage 130.

In FIG. 1, although an example is described in which learner 140 of server device 100 generates a pre-trained person detection AI 132 (a so-called person detection AI model), and person detection AI 132 is updated by storing the pre-trained person detection AI 132 in storage 130, this example is not limiting. For example, a pre-trained person detection AI 132 may be generated in a cloud server provided outside of the building, and the cloud server may update person detection AI 132 by transmitting the pre-trained person detection AI 132 to server device 100.

Although an example is described in which supervised learning, among the methods of machine learning, is performed in learner 140, machine learning including unsupervised learning, reinforced learning, deep learning, and the like may be performed. Furthermore, in the learning performed in person detection AI 132, learning that uses AI learning other than that described in the above-mentioned machine learning methods may be applied as needed.

### [2. Operation Example]

Next, operation of human body surface temperature calculation system 200 will be described. FIG. 2 is a flowchart illustrating an operation example of human body surface temperature calculation system 200. FIG. 3 is a diagram schematically illustrating the flow illustrated in FIG. 2.

Communicator 110 of server device 100 receives temperature distribution data from infrared sensor 10 (not illustrated in the figure). Here, information processor 120 stores the temperature distribution data received to storage 130 (not illustrated in the figure).

Next, obtainer 121 obtains the temperature distribution data that is received by communicator 110 and stored in storage 130 (step S01), and outputs the temperature distribution data to thermal image generator 122. Thermal image generator 122 generates a thermal image of the target space based on the temperature distribution data obtained (thermal image generation step S02). For example, as illustrated in (a) in FIG. 3, the temperature distribution data obtained by obtainer 121 in step S01 is a temperature value matrix. Furthermore, as illustrated in (b) in FIG. 3, the thermal image generated by thermal image generator 122 in thermal image generation step S02 is, for example, an 8-bit image in which a temperature range from 20°C to 40°C has been converted to a 0 to 255 scale image.

As illustrated in (c) in FIG. 3, thermal image generator 122 may resize the thermal image generated, and may perform image conversion processing, such as contrast enhancement and negative-positive inversion, and the like. Accordingly, a person detection AI, such as an object detection AI and a posture estimation AI applied to RGB images can be applied to the thermal image.

Next, identifier 123 detects a person visible in the thermal image using person detection AI 132, and identifies a person detection area that is a rectangular area that includes the person (identifying step S03). More specifically, as illustrated in (d) in FIG. 3, identifier 123 identifies person detection area A1 that includes the person detected in the thermal image using person detection AI 132.

Next, first calculator 124 extracts a first temperature value group corresponding to person detection area A1 (see (d) in FIG. 3) identified in step S03 from the temperature distribution data (step S04; (e) in FIG. 3), and calculates, based on the first temperature value group, a temperature of background area B1 included in person detection area A1, in which a background excluding the person is visible, and an average temperature of person detection area A1 (step S05; (f) in FIG. 3). The first calculation step is a step that includes step S04 and step S05. The specific processes of the first calculation step will be described later.

Next, second calculator 125 extracts a second temperature value group from the first temperature value group based on a rule determined by a magnitude relationship between the temperature of background area B1 and the average temperature of person detection area A1 calculated in step S05 (step S06; (g) in FIG. 3), and calculates a surface temperature of the person based on the second temperature value group (step S07; (h) in FIG. 3). The second calculation step is a step that includes step S06 and step S07. It should be noted that the extraction method of the second temperature value group performed in step S06 is referred to as a first method. The first method will be described later in detail. In step S07, second calculator 125 may extract temperature values (so-called second temperature values) corresponding to pixels in person area C1 in the thermal image from a matrix of the temperature distribution data, and may calculate a surface temperature of the person by calculating an average value or a median value of the temperature value group (so-called second temperature value group) extracted.

Next, second calculator 125 outputs a calculation result (not illustrated in the figure). Specifically, second calculator 125 outputs a surface temperature of a person present in the target space as a calculation result. In addition to a surface temperature of a person, second calculator 125 may output information indicating coordinates of the person in the target space as a calculation result. It should be noted that the calculation result is stored in storage 130.

As described above, human body surface temperature calculation system 200 can detect that a person is present in a target space, calculate a surface temperature of the person detected, and output a calculation result.

The calculation result outputted may be provided to a control device (not illustrated in the figures) that controls a device, such as an air conditioner and the like, for example. Accordingly, the control device can control the device based on the surface temperature of the person in the target space.

### [Specific Example of First Calculation Step]

Next, the specific processes of the first calculation step will be described with reference to the drawings.

### [First Example]

In the first example, a processing example will be described of a case where an area in which a person is shown (person area) overlaps with one corner among four corners of a person detection area that includes a person visible in a thermal image. FIG. 4 is a diagram illustrating an example of a thermal image. FIG. 5 is a diagram for describing a first example of a process for a first calculation step.

From among four temperature values that correspond to four corners, namely, P1, P2, P3, and P4 of person detection area A2 included in a first temperature value group that corresponds to person detection area A2 (see FIG. 4) that includes the person visible in the thermal image, first calculator 124 calculates, as a temperature of background area B2, an average value of two temperature values (temperature values of P1 and P3, for example) obtained by excluding a largest temperature value (temperature value of P4, for example) and a smallest temperature value (temperature value of P2, for example). Background area B2 is an area in which a background excluding the person visible in the thermal image is shown. The area in which the person visible in the thermal image is shown is person area C2.

As described above, in human body surface temperature calculation system 200, since a largest temperature value and a smallest temperature value are excluded from the four temperature values that each correspond to one of the four corners of person detection area A2, in a case where there is an abnormally large value or an abnormally small value, the influence of such values can thus be eliminated. Accordingly, since human body surface temperature calculation system 200 can accurately calculate the temperature of background area B2, human body surface temperature calculation system 200 can accurately calculate the surface temperature of the person (average temperature of person area C2, for example).

### [Second Example]

In the second example, a processing example will be described of a case in which an area in which a person is shown (person area) overlaps with two or more corners among four corners of a person detection area that includes a person visible in a thermal image. FIG. 6 is a diagram for describing a second example of a process for the first calculation step.

From among four temperature values that correspond to four corners, namely, P1, P2, P3, and P4 of person detection area A3 included in a first temperature value group that corresponds to person detection area A3 that includes the person visible in the thermal image, first calculator 124 calculates, as a temperature of background area B3, an average value of two temperature values (temperature values of P1 and P2, for example) obtained by excluding the top two temperature values (temperature values of P3 and P4, for example) having the largest and the second largest absolute value differences from a temperature value of a central portion of person detection area A3. Background area B3 is an area in which a background excluding the person visible in the thermal image is shown. The area in which the person visible in the thermal image is shown is person area C3.

As described above, when two corners among the four corners of person detection area A3 overlap with person area C3, since the two temperature values that correspond to the overlapping two corners can be excluded from the calculation of a temperature of background area B3, human body surface temperature calculation system 200 can thereby accurately calculate the temperature of background area B3. Accordingly, since human body surface temperature calculation system 200 can accurately calculate a temperature of background area B3, even when the person occupies a large portion of the thermal image, human body surface temperature calculation system 200 can accurately calculate a surface temperature of the person (average temperature of person area C3, for example).

### [Specific Example of Second Calculation Step]

Next, the specific processes of the second calculation step will be described with reference to the drawings.

### [First Example]

In the first example, a processing example will be described in which second calculator 125 extracts a second temperature value group from a first temperature value group by a first method, and calculates a surface temperature of a person based on the second temperature value group extracted. It should be noted that the first method is the extraction method of the second temperature value group performed in step S06 in FIG. 2. FIG. 7 is a flowchart illustrating an example of a detailed flow of step S06 in FIG. 2.

Second calculator 125 determines whether the average temperature of the person detection area calculated in step S05 in FIG. 2 is higher than a temperature of a background area (step S11). When the average temperature of the person detection area is higher than the temperature of the background area ("Yes" in step S11), second calculator 125 calculates a first value (step S12). The first value is a value obtained by subtracting predetermined value α from a largest temperature value included in the first temperature value group. Next, second calculator 125 extracts, as a second temperature value group, a temperature value group that is a group of temperature values that are included in the first temperature value group and are each greater than or equal to the first value (step S13). For example, during summer, when a person enters a room from outside, a surface temperature of the person will be higher than an indoor temperature. For example, when the indoor temperature is 26°C and a largest value of surface temperatures of a person is 36°C, and where predetermined value α is 4°C, the first value is 32°C. In this case, second calculator 125 extracts, as a second temperature value group, a temperature value group that is a group of temperature values that are included in the first temperature value group and are each greater than or equal to 32°C. It should be noted that a predetermined value α may be determined for each indoor temperature, and the value may be determined by way of experiment or based on experience.

On the other hand, when the average temperature of the person detection area is lower than the temperature of the background area ("No" in step S11), second calculator 125 calculates a second value (step S14). The second value is a value obtained by adding predetermined value β to a smallest temperature value included in the first temperature value group. Next, second calculator 125 extracts, as a second temperature value group, a temperature value group that is a group of temperature values that are included in the first temperature value group and are each less than or equal to the second value (step S15). For example, during winter, when a person enters a room from outside, a surface temperature of the person will be lower than an indoor temperature. For example, when the indoor temperature is 20°C and a smallest value of surface temperatures of a person is 4°C, and where predetermined value β is 10°C, the second value is 14°C. In this case, second calculator 125 extracts, as a second temperature value group, a temperature value group that is a group of temperature values that are each less than or equal to 14°C. It should be noted that a predetermined value β may be determined for each indoor temperature, and the value may be determined by way of experiment or based on experience.

Second calculator 125 calculates a surface temperature of a person based on the second temperature value group extracted in step S13 or step S15 (step S07 in FIG. 2).

Note that when the average value of the person detection area is the same as the temperature of the background area, either of the above-mentioned methods can be used to extract the second temperature value group.

As described above, according to the first method, for example, human body surface temperature calculation system 200 can accurately extract a second temperature value group from a first temperature value group even when a person enters a warm room from a cold outdoor environment during winter, or when a person enters a cool room from a hot outdoor environment during summer. Consequently, according to the first method, human body surface temperature calculation system 200 can accurately calculate a surface temperature of a person even in a state where the surface temperature (surface temperature of clothing in particular) of the person is influenced by outside temperatures.

### [Second Example]

In the second example, a processing example will be described in which second calculator 125 extracts a second temperature value group from a first temperature value group by a second method, and calculates a surface temperature of a person based on the second temperature value group extracted. The second method may be performed in a case in which a posture estimation AI is used as a person detection AI. FIG. 8 is a diagram illustrating a person detection area detected in a thermal image by a posture estimating AI. FIG. 9 is a diagram illustrating an example of an area defined by lines connecting different nodes in a skeletal frame model of a body of the person.

Before describing the second method, a method for identifying a person detection area in a thermal image using a posture estimation AI as a person detection AI will be described.

In step S02 in FIG. 2, thermal image generator 122 generates a thermal image of a target space, and resizes the thermal image generated, and performs image conversion processing, such as contrast enhancement and negative-positive inversion, and the like. Accordingly, a person detection AI, such as an object detection AI and a posture estimation AI applied to RGB images can be applied to the thermal image.

Next, in step S03 in FIG. 2, identifier 123 identifies a person detection area that is a rectangular area that includes a person visible in the thermal image using a posture estimation AI as person detection AI 132. Specifically, for example, as illustrated in FIG. 8, identifier 123 identifies, as person detection area A4, a rectangular area that is surrounded by lines that connect, from among a plurality of nodes (so-called feature points; indicated as black dots in the figures) included in a skeletal frame model of the body of the person detected in the thermal image by the posture estimation AI, outermost nodes (nodes indicating both wrists, nodes indicating both ankles, and nodes indicating both eyes, for example) for the up, down, left, and right directions of the thermal image. For example, identifier 123 may estimate the position of the top of the head of the person based on a positional relationship between nodes indicating both eyes, both ears, and the nose, and the like of the person, and may identify a rectangular area that includes everything from the top of the head to the ankles of the person as person detection area A4.

Next, first calculator 124 extracts a first temperature value group corresponding to the person detection area A4 identified by identifier 123 from the temperature distribution data (step S04 in FIG. 2). The second example is different from the first example in that first calculator 124 does not execute the process in step S05 in FIG. 2. Specifically, first calculator 124 need not calculate a temperature of a background area included in person detection area A4, in which a background excluding the person is visible, and it need not calculate an average temperature of person detection area A4 based on the first temperature value group.

Next, second calculator 125 extracts a second temperature value group from the first temperature value group by the second method. Specifically, for example, as illustrated in FIG. 9, second calculator 125 extracts, as a second temperature value group, a temperature value group that is a group of temperature values that are included in the first temperature value group extracted by first calculator 124 and are included in an area (a so-called person area C4) defined by lines connecting different nodes in a skeletal frame model of the body of the person detected in the thermal image by a posture estimation AI. For example, second calculator 125 may identify, as a person area, an area having predetermined widths that is symmetrical relative to lines that connect different nodes in the skeletal frame model of the body of the person. In this case, predetermined widths may each be set for parts of the body, such as arms, thighs, calves, and the like. Furthermore, for a torso portion that is surrounded by lines connecting different nodes in the skeletal frame model of the person, second calculator 125 may identify, as the torso portion of the person, an area defined by the lines indicating the torso portion that further includes an area that is covered when the lines are enlarged outward by a predetermined width.

Next, second calculator 125 calculates a surface temperature of the person based on the second temperature value group extracted by the second method.

As described above, according to the second method, human body surface temperature calculation system 200 can extract the second temperature value group in a manner that closely matches an outline of the person regardless of a temperature of the background area or a difference between the temperature of the background area and a threshold value, or the like. Therefore, according to the second method, calculation costs can be reduced since human body surface temperature calculation system 200 can extract a second temperature value group corresponding to a person area based on a skeletal frame model of a person detected by a posture estimation AI, without the need to take into consideration seasonal differences, for seasons such as summer and winter, in temperatures of background areas and average temperatures of person detection areas.

### [3. Advantageous Effects, etc.]

As described above, human body surface temperature calculation system 200 includes: thermal image generator 122 that generates a thermal image of a target space based on temperature distribution data indicating a temperature distribution of the target space obtained by infrared sensor 10; identifier 123 that identifies person detection area A1 using a person detection artificial intelligence (AI) 132, person detection area A1 being a rectangular area that includes a person visible in the thermal image (see FIG. 3); first calculator 124 that extracts a first temperature value group corresponding to person detection area A1 from the temperature distribution data, and calculates, based on the first temperature value group extracted, a temperature of background area B1 and an average temperature of person detection area A1, background area B1 being an area that is included in person detection area A1 and in which a background excluding the person is visible (see FIG. 3); and second calculator 125 that extracts a second temperature value group from the first temperature value group based on a rule determined by a magnitude relationship between the temperature of background area B1 calculated and the average temperature of person detection area A1 calculated, and calculates a surface temperature of the person based on the second temperature value group extracted.

Since such a human body surface temperature calculation system 200 applies a rule determined by a magnitude relationship between a temperature of the target space (background temperature of the thermal image) and a surface temperature of the person, human body surface temperature calculation system 200 can accurately extract the second temperature value group even in a case where the surface temperature of the person is influenced by an outside temperature, such as in summer and in winter, for example. Consequently, human body surface temperature calculation system 200 can accurately calculate the surface temperature of the person present in the target space.

Furthermore, for example, first calculator 124 calculates, as the temperature of background area B2 (see FIG. 5), an average value of two temperature values (temperature values P1 and P3, for example) obtained by excluding a largest temperature value (temperature value P4, for example) and a smallest temperature value (temperature value P2, for example) from four temperature values P1, P2, P3, and P4 that correspond to four corners of person detection area A2 (see FIG. 4) and are included in the first temperature value group that corresponds to person detection area A2 that includes the person visible in the thermal image (see FIG. 4).

In such a human body surface temperature calculation system 200, by excluding the largest value and the smallest value, from among four temperature values that each correspond to one of the four corners of person detection area A2, in a case where there is an abnormally large value or an abnormally small value, the influence of such values can thus be eliminated. Accordingly, since human body surface temperature calculation system 200 can accurately calculate the temperature of background area B2, human body surface temperature calculation system 200 can accurately calculate the surface temperature of the person (average temperature of person area C2, for example).

Furthermore, for example, first calculator 124 calculates, as the temperature of background area B3, an average value of two temperature values (temperature values P1 and P2, for example) obtained by excluding top two temperature values (temperature values P3 and P4, for example) from four temperature values P1, P2, P3, and P4 that correspond to four corners of person detection area A3 and are included in the first temperature value group that corresponds to person detection area A3 that includes the person visible in the thermal image (see FIG. 6), the top two temperature values having largest and second largest absolute value differences from a temperature value of a central portion of person detection area A3. Background area B3 is an area in which a background excluding the person visible in the thermal image is shown. The area in which the person visible in the thermal image is shown is person area C3.

In such a human body surface temperature calculation system 200, when two corners among the four corners of person detection area A3 overlap with person area C3, since the two temperature values that correspond to the overlapping two corners can be excluded from the calculation of the temperature of background area B3, human body surface temperature calculation system 200 can thereby accurately calculate the temperature of background area B3. Accordingly, since human body surface temperature calculation system 200 can accurately calculate the temperature of background area B3, even when the person occupies a large portion of the thermal image, human body surface temperature calculation system 200 can accurately calculate the surface temperature of the person (average temperature of person area C3, for example).

Furthermore, for example, when the average temperature of the person detection area is higher than the temperature of the background area ("Yes" in step S11 in FIG. 7), the second temperature value group is a group of temperature values that are included in the first temperature value group and are each greater than or equal to a first value (step S13 in FIG. 7), the first value being a value obtained by subtracting a predetermined value α from a largest temperature value included in the first temperature value group, and when the average temperature of the person detection area is lower than the temperature of the background area ("No" in step S11 in FIG. 7), the second temperature value group is a group of temperature values that are included in the first temperature value group and are each less than or equal to a second value (step S15 in FIG. 7), the second value being a value obtained by adding a predetermined value β to a smallest temperature value included in the first temperature value group. In human body surface temperature calculation system 200, the second temperature value group is extracted by a first method in which the second temperature value group is extracted from the first temperature value group based on the above-mentioned rule determined by the magnitude relationship between the temperature of the background area and the average temperature of the person detection area.

In such a human body surface temperature calculation system 200, according to the first method, for example, the second temperature value group can be accurately extracted from the first temperature value group even when a person enters a warm room from a cold outdoor environment during winter, or when a person enters a cool room from a hot outdoor environment during summer. Consequently, in human body surface temperature calculation system 200, according to the first method, the surface temperature of the person can be accurately calculated even when the surface temperature of the person (surface temperature of clothing in particular) is influenced by an outside temperature.

Furthermore, for example, person detection AI 132 is an object detection AI.

Such a human body surface temperature calculation system 200 can detect a person visible in the thermal image using an object detection AI.

Furthermore, for example, person detection AI 132 is a posture estimation AI.

Such a human body surface temperature calculation system 200 can detect a person visible in the thermal image using a posture estimation AI.

Furthermore, for example, person detection area A4 (see FIG. 8) is a rectangular area surrounded by lines that connect, from among a plurality of nodes included in a skeletal frame model of a body of the person detected in the thermal image by the posture estimation AI, outermost nodes for up, down, left, and right directions of the thermal image.

Such a human body surface temperature calculation system 200 can identify the person detection area from information included in the skeletal frame model of the body of the person detected using the posture estimation AI.

Furthermore, for example, in addition to a first method for extracting the second temperature value group based on the rule, second calculator 125 is capable of extracting the second temperature value group by a second method, and in the second method, second calculator 125 extracts, as the second temperature value group, a temperature value group, from the first temperature value group, the temperature value group being included in an area (so-called person area C4) defined by lines connecting different nodes in a skeletal frame model of a body of the person detected in the thermal image by the posture estimation AI.

According to the second method, such a human body surface temperature calculation system 200 can extract the second temperature value group in a manner that closely matches an outline of the person regardless of the temperature of the background area or a difference between the temperature of the background area and a threshold value, or the like. Consequently, according to the second method, calculation costs can be reduced since human body surface temperature calculation system 200 can extract a second temperature value group corresponding to a person area based on the skeletal frame model of the person detected by the posture estimation AI, without the need to take into consideration seasonal differences, for seasons such as summer and winter, in the average temperatures of background areas and person detection areas.

Furthermore, a human body surface temperature calculation method executed by a computer, such as human body surface temperature calculation system 200 is a human body surface temperature calculation method that includes: generating a thermal image of a target space based on temperature distribution data indicating a temperature distribution of the target space obtained by infrared sensor 10 (step S02 in FIG. 2); identifying person detection area A1 (see FIG. 3) using person detection artificial intelligence (AI) 132, person detection area A1 being a rectangular area that includes a person visible in the thermal image (step S03); extracting a first temperature value group corresponding to person detection area A1 from the temperature distribution data, and calculating, based on the first temperature value group extracted, a temperature of background area B2 and an average temperature of person detection area A1, background area B2 being an area that is included in person detection area A1 and in which a background excluding the person is visible (step S04 and step S05); and extracting a second temperature value group from the first temperature value group based on a rule determined by a magnitude relationship between the temperature of background area B1 calculated (see FIG. 3) and the average temperature of person detection area A1 calculated, and calculating a surface temperature of the person based on the second temperature value group extracted (step S06 and step S07).

Since such a human body surface temperature calculation method applies a rule determined by a magnitude relationship between a temperature of the target space (background temperature of the thermal image) and a surface temperature of the person, the human body surface temperature calculation method can accurately extract the second temperature value group even in a case where the surface temperature of the person is influenced by an outside temperature, such as in summer and in winter, for example. Consequently, the human body surface temperature calculation method can accurately calculate the surface temperature of the person present in the target space.

### (Other Embodiments)

Although human body surface temperature calculation system 200 and the human body surface temperature calculation method according to an embodiment have been described above, the present invention is not limited to the above embodiment.

For example, in the above-mentioned embodiment, although an example is described in which identifier 123 uses an object detection AI or a posture estimation AI as person detection AI 132, identifier 123 may use both an object detection AI and a posture estimation AI as person detection AI 132.

Furthermore, in the above-mentioned embodiment, although an example is described in which human body surface temperature calculation system 200 includes one infrared sensor 10 provided in one target space, human body surface temperature calculation system 200 may include a plurality of infrared sensors 10 provided in one target space, and may include a plurality of infrared sensors 10 provided in a plurality of target spaces.

Furthermore, the steps described as an example in the above-mentioned embodiment may be combined as needed to calculate a surface temperature of a person.

Furthermore, in the above-mentioned embodiment, although an example is described in which communicator 110 is a communication module (or communication circuit) for communicating with infrared sensor 10, and that wireless communication and wired communication may be performed for communication by communicator 110, this example is not limiting. For example, communication from infrared sensor 10 to server device 100 may be in a format in which analog output from infrared sensor 10 is input to an A/D input port of a microcomputer, and may be in a format in which an infrared image (a so-called, thermal image) is obtained as input from an RGB camera from infrared sensor 10 (an infrared camera, for example) via a USB cable.

Furthermore, in the above-mentioned embodiment, human body surface temperature calculation system 200 is implemented as a plurality of devices, but may be implemented as a single device. For example, human body surface temperature calculation system 200 may be implemented as a single device equivalent to the server device. When implementing human body surface temperature calculation system 200 as a plurality of devices, each element included in human body surface temperature calculation system 200 may be assigned to the plurality of devices in any manner.

Furthermore, in the above-mentioned embodiment, processes to be executed by a given processor may be performed by another processor. Furthermore, the order of the plurality of processes may be changed, and the plurality of processes may be executed in parallel with each other.

Furthermore, in the above-mentioned embodiment, each element may be implemented by executing a software program suitable for each element. The respective elements may be implemented by a program executer, such as a CPU or a processor, reading and executing a software program recorded on a recording medium, such as a hard disk or a semiconductor memory.

Furthermore, each element may be implemented as hardware. For example, each element may be a circuit (or an integrated circuit). Such circuits may be consolidated as a single circuit, and may be configured as individual circuits. Furthermore, such circuits may each be a general-purpose circuit or a dedicated circuit.

Furthermore, general and specific aspects of the present invention may be implemented as a system, a device, a method, an integrated circuit, a computer program, or a computer-readable recording medium such as a CD-ROM. Furthermore, general and specific aspects of the present invention may be implemented as any combination of a system, a device, a method, an integrated circuit, a computer program, or a recording medium. For example, the present invention may be implemented as a human body surface temperature calculation method executed by a computer, such as a human body surface temperature calculation system. Furthermore, the present invention may be implemented as a program for causing a computer to execute the human body surface temperature calculation method, and may be implemented as a non-transitory computer-readable recording medium having recorded thereon such a program.

Forms obtained through various modifications to the foregoing embodiments that can be conceived by those skilled in the art, as well as forms realized by combining elements and functions in the foregoing embodiments without departing from the essence of the present invention are included within the scope of the present invention.

### [Reference Signs List]

- 10: infrared sensor
- 122: thermal image generator
- 123: identifier
- 124: first calculator
- 125: second calculator
- 132: person detection AI
- 200: human body surface temperature calculation system
- A1, A2, A3, A4: person detection area
- B1, B2, B3, B4: background area

## Claims

1. A human body surface temperature calculation system comprising:
a thermal image generator that generates a thermal image of a target space based on temperature distribution data indicating a temperature distribution of the target space obtained by an infrared sensor;
an identifier that identifies a person detection area using a person detection artificial intelligence (AI), the person detection area being a rectangular area that includes a person visible in the thermal image;
a first calculator that extracts a first temperature value group corresponding to the person detection area from the temperature distribution data, and calculates, based on the first temperature value group extracted, a temperature of a background area and an average temperature of the person detection area, the background area being an area that is included in the person detection area and in which a background excluding the person is visible; and
a second calculator that extracts a second temperature value group from the first temperature value group based on a rule determined by a magnitude relationship between the temperature of the background area calculated and the average temperature of the person detection area calculated, and calculates a surface temperature of the person based on the second temperature value group extracted.

2. The human body surface temperature calculation system according to claim 1, wherein
the first calculator calculates, as the temperature of the background area, an average value of two temperature values obtained by excluding a largest temperature value and a smallest temperature value from four temperature values that correspond to four corners of the person detection area and are included in the first temperature value group.

3. The human body surface temperature calculation system according to claim 1, wherein
the first calculator calculates, as the temperature of the background area, an average value of two temperature values obtained by excluding top two temperature values from four temperature values that correspond to four corners of the person detection area and are included in the first temperature value group, the top two temperature values having largest and second largest absolute value differences from a temperature value of a central portion of the person detection area.

4. The human body surface temperature calculation system according to any one of claims 1 to 3, wherein
when the average temperature of the person detection area is higher than the temperature of the background area, the second temperature value group is a group of temperature values that are included in the first temperature value group and are each greater than or equal to a first value, the first value being a value obtained by subtracting a predetermined value α from a largest temperature value included in the first temperature value group, and
when the average temperature of the person detection area is lower than the temperature of the background area, the second temperature value group is a group of temperature values that are included in the first temperature value group and are each less than or equal to a second value, the second value being a value obtained by adding a predetermined value β to a smallest temperature value included in the first temperature value group.

5. The human body surface temperature calculation system according to any one of claims 1 to 3, wherein
the person detection AI is an object detection AI.

6. The human body surface temperature calculation system according to any one of claims 1 to 3, wherein
the person detection AI is a posture estimation AI.

7. The human body surface temperature calculation system according to claim 6, wherein
the person detection area is a rectangular area surrounded by lines that connect, from among a plurality of nodes included in a skeletal frame model of a body of the person detected in the thermal image by the posture estimation AI, outermost nodes for up, down, left, and right directions of the thermal image.

8. The human body surface temperature calculation system according to claim 6, wherein
in addition to a first method for extracting the second temperature value group based on the rule, the second calculator is capable of extracting the second temperature value group by a second method, and
in the second method, the second calculator extracts, as the second temperature value group, a temperature value group, from the first temperature value group, the temperature value group being included in an area defined by lines connecting different nodes in a skeletal frame model of a body of the person detected in the thermal image by the posture estimation AI.

9. A human body surface temperature calculation method executed by a computer, the human body surface temperature calculation method comprising:
generating a thermal image of a target space based on temperature distribution data indicating a temperature distribution of the target space obtained by an infrared sensor;
identifying a person detection area using a person detection artificial intelligence (AI), the person detection area being a rectangular area that includes a person visible in the thermal image;
extracting a first temperature value group corresponding to the person detection area from the temperature distribution data, and calculating, based on the first temperature value group extracted, a temperature of a background area and an average temperature of the person detection area, the background area being an area that is included in the person detection area and in which a background excluding the person is visible; and
extracting a second temperature value group from the first temperature value group based on a rule determined by a magnitude relationship between the temperature of the background area calculated and the average temperature of the person detection area calculated, and calculating a surface temperature of the person based on the second temperature value group extracted.

10. A program for causing the computer to execute the human body surface temperature calculation method according to claim 9.

## Patentansprüche

1. System zur Berechnung der Oberflächentemperatur des menschlichen Körpers, umfassend:
einen Wärmebildgenerator, der ein Wärmebild eines Zielraums auf der Grundlage von Temperaturverteilungsdaten erzeugt, die eine Temperaturverteilung des Zielraums angeben, die durch einen Infrarotsensor erhalten ist;
eine Identifizierungsvorrichtung, die unter Verwendung einer künstlichen Intelligenz (KI) zur Personenerkennung einen Personenerkennungsbereich identifiziert, wobei der Personenerkennungsbereich ein rechteckiger Bereich ist, der eine in dem Wärmebild sichtbare Person enthält;
einen ersten Rechner, der aus den Temperaturverteilungsdaten eine erste Temperaturwertgruppe extrahiert, die dem Personenerkennungsbereich entspricht, und auf der Grundlage der extrahierten ersten Temperaturwertgruppe eine Temperatur eines Hintergrundbereichs und eine Durchschnittstemperatur des Personenerkennungsbereichs berechnet, wobei der Hintergrundbereich ein Bereich ist, der in dem Personenerkennungsbereich enthalten ist und in dem ein die Person ausschließender Hintergrund sichtbar ist; und
einen zweiten Rechner, der auf der Grundlage einer Regel, die durch ein Größenverhältnis zwischen der berechneten Temperatur des Hintergrundbereichs und der berechneten Durchschnittstemperatur des Personenerkennungsbereichs bestimmt wird, eine zweite Temperaturwertgruppe aus der ersten Temperaturwertgruppe extrahiert und auf der Grundlage der extrahierten zweiten Temperaturwertgruppe eine Oberflächentemperatur der Person berechnet.

2. System zur Berechnung der Oberflächentemperatur des menschlichen Körpers nach Anspruch 1, wobei
der erste Rechner als die Temperatur des Hintergrundbereichs einen Durchschnittswert aus zwei Temperaturwerten berechnet, die durch Ausschließen eines größten Temperaturwerts und eines kleinsten Temperaturwerts aus vier Temperaturwerten erhalten werden, die vier Ecken des Personenerkennungsbereichs entsprechen und in der ersten Temperaturwertgruppe enthalten sind.

3. System zur Berechnung der Oberflächentemperatur des menschlichen Körpers nach Anspruch 1, wobei
der erste Rechner als Temperatur des Hintergrundbereichs einen Durchschnittswert aus zwei Temperaturwerten berechnet, die durch Ausschließen der beiden höchsten Temperaturwerte aus vier Temperaturwerten erhalten werden, die vier Ecken des Personenerkennungsbereichs entsprechen und in der ersten Temperaturwertgruppe enthalten sind, wobei die beiden höchsten Temperaturwerte die größten und zweitgrößten absoluten Differenzwerte zu einem Temperaturwert eines zentralen Abschnitts des Personenerkennungsbereichs aufweisen.

4. System zur Berechnung der Oberflächentemperatur des menschlichen Körpers nach einem der Ansprüche 1 bis 3, wobei
wenn die Durchschnittstemperatur des Personenerkennungsbereichs höher ist als die Temperatur des Hintergrundbereichs, die zweite Temperaturwertgruppe eine Gruppe von Temperaturwerten ist, die in der ersten Temperaturwertgruppe enthalten sind und jeweils größer oder gleich einem ersten Wert sind, wobei der erste Wert ein Wert ist, der durch Subtrahieren eines vorbestimmten Werts α von einem größten Temperaturwert erhalten wird, der in der ersten Temperaturwertgruppe enthalten ist, und
wenn die Durchschnittstemperatur des Personenerkennungsbereichs niedriger ist als die Temperatur des Hintergrundbereichs, die zweite Temperaturwertgruppe eine Gruppe von Temperaturwerten ist, die in der ersten Temperaturwertgruppe enthalten sind und jeweils kleiner oder gleich einem zweiten Wert sind, wobei der zweite Wert ein Wert ist, der durch Addieren eines vorbestimmten Werts β zu einem kleinsten Temperaturwert erhalten wird, der in der ersten Temperaturwertgruppe enthalten ist.

5. System zur Berechnung der Oberflächentemperatur des menschlichen Körpers nach einem der Ansprüche 1 bis 3, wobei
die Personenerkennungs-KI eine Objekterkennungs-KI ist.

6. System zur Berechnung der Oberflächentemperatur des menschlichen Körpers nach einem der Ansprüche 1 bis 3, wobei
die Personenerkennungs-KI eine Haltungsschätzungs-KI ist.

7. System zur Berechnung der Oberflächentemperatur des menschlichen Körpers nach Anspruch 6, wobei
der Personenerkennungsbereich ein rechteckiger Bereich ist, der von Linien umgeben ist, die unter einer Vielzahl von Knoten, die in einem Skelettrahmenmodell des Körpers der durch die Haltungsschätzungs-KI in dem Wärmebild erkannten Person enthalten sind, äußerste Knoten für Richtungen nach oben, unten, links und rechts des Wärmebildes verbinden.

8. System zur Berechnung der Oberflächentemperatur des menschlichen Körpers nach Anspruch 6, wobei
zusätzlich zu einem ersten Verfahren zum Extrahieren der zweiten Temperaturwertgruppe auf der Grundlage der Regel der zweite Rechner in der Lage ist, die zweite Temperaturwertgruppe durch ein zweites Verfahren zu extrahieren, und
bei dem zweiten Verfahren der zweite Rechner als zweite Temperaturwertgruppe eine Temperaturwertgruppe aus der ersten Temperaturwertgruppe extrahiert, wobei die Temperaturwertgruppe in einem Bereich enthalten ist, der durch Linien definiert ist, die unterschiedliche Knoten in einem Skelettrahmenmodell des Körpers der in dem Wärmebild durch die Haltungsschätzungs-KI erkannten Person verbinden.

9. Verfahren zur Berechnung der Oberflächentemperatur des menschlichen Körpers, das von einem Computer ausgeführt wird, wobei das Verfahren zur Berechnung der Oberflächentemperatur des menschlichen Körpers umfasst:
Erzeugen eines Wärmebildes eines Zielraums auf der Grundlage von Temperaturverteilungsdaten, die eine Temperaturverteilung des Zielraums angeben, die durch einen Infrarotsensor erhalten wird;
Identifizieren eines Personenerkennungsbereichs unter Verwendung einer künstlichen Intelligenz (KI) zur Personenerkennung, wobei der Personenerkennungsbereich ein rechteckiger Bereich ist, der eine in dem Wärmebild sichtbare Person enthält;
Extrahieren einer ersten Temperaturwertgruppe, die dem Personenerkennungsbereich entspricht, aus den Temperaturverteilungsdaten und Berechnen einer Temperatur eines Hintergrundbereichs und einer Durchschnittstemperatur des Personenerkennungsbereichs auf der Grundlage der extrahierten ersten Temperaturwertgruppe, wobei der Hintergrundbereich ein Bereich ist, der in dem Personenerkennungsbereich enthalten ist und in dem ein die Person ausschließender Hintergrund sichtbar ist; und
Extrahieren einer zweiten Temperaturwertgruppe aus der ersten Temperaturwertgruppe auf der Grundlage einer Regel, die durch ein Größenverhältnis zwischen der berechneten Temperatur des Hintergrundbereichs und der berechneten Durchschnittstemperatur des Personenerkennungsbereichs bestimmt wird, und Berechnen einer Oberflächentemperatur der Person auf der Grundlage der extrahierten zweiten Temperaturwertgruppe.

10. Programm, das den Computer veranlasst, das Verfahren zur Berechnung der Oberflächentemperatur des menschlichen Körpers nach Anspruch 9 auszuführen.

## Revendications

1. Un système de calcul de la température de surface du corps humain comprenant :
un générateur d'image thermique qui effectue la génération d'une image thermique d'un espace cible sur la base de données de distribution de température indiquant une distribution de température de l'espace cible obtenue par un capteur infrarouge ;
un identificateur qui effectue l'identification d'une zone de détection de personne à l'aide d'une intelligence artificielle de détection de personne (IA), la zone de détection de personne étant une zone rectangulaire qui comprend une personne visible dans l'image thermique ;
un premier calculateur qui effectue l'extraction d'un premier groupe de valeurs de température correspondant à la zone de détection de personne à partir des données de distribution de température, et effectue le calcul, sur la base du premier groupe de valeurs de température extrait, d'une température d'une zone d'arrière-plan et d'une température moyenne de la zone de détection de personne, la zone d'arrière-plan étant une zone qui est incluse dans la zone de détection de personne et dans laquelle un arrière-plan à l'exclusion de la personne est visible ; et
un second calculateur qui effectue l'extraction d'un second groupe de valeurs de température à partir du premier groupe de valeurs de température sur la base d'une règle déterminée par une relation de grandeur entre la température de la zone d'arrière-plan calculée et la température moyenne de la zone de détection de personne calculée, et effectue le calcul d'une température de surface de la personne sur la base du second groupe de valeurs de température extrait.

2. Le système de calcul de la température de surface du corps humain selon la revendication 1, dans lequel
le premier calculateur effectue le calcul, en tant que température de la zone d'arrière-plan, d'une valeur moyenne de deux valeurs de température obtenues par l'exclusion d'une valeur de température la plus grande et d'une valeur de température la plus petite à partir de quatre valeurs de température qui correspondent à quatre coins de la zone de détection de personne et sont incluses dans le premier groupe de valeurs de température.

3. Le système de calcul de la température de surface du corps humain selon la revendication 1, dans lequel
le premier calculateur effectue le calcul, en tant que température de la zone d'arrière-plan, d'une valeur moyenne de deux valeurs de température obtenues par l'exclusion des deux valeurs de température les plus élevées à partir de quatre valeurs de température qui correspondent à quatre coins de la zone de détection de personne et sont incluses dans le premier groupe de valeurs de température, les deux valeurs de température les plus élevées ayant les différences de valeur absolue les plus grande et deuxième plus grande par rapport à une valeur de température d'une partie centrale de la zone de détection de personne.

4. Le système de calcul de la température de surface du corps humain selon l'une quelconque des revendications 1 à 3, dans lequel
lorsque la température moyenne de la zone de détection de personne est supérieure à la température de la zone d'arrière-plan, le second groupe de valeurs de température est un groupe de valeurs de température qui sont incluses dans le premier groupe de valeurs de température et sont chacune supérieure ou égale à une première valeur, la première valeur étant une valeur obtenue par la soustraction d'une valeur prédéterminée α à partir d'une valeur de température la plus grande incluse dans le premier groupe de valeurs de température, et
lorsque la température moyenne de la zone de détection de personne est inférieure à la température de la zone d'arrière-plan, le second groupe de valeurs de température est un groupe de valeurs de température qui sont incluses dans le premier groupe de valeurs de température et sont chacune inférieure ou égale à une seconde valeur, la seconde valeur étant une valeur obtenue par l'addition d'une valeur prédéterminée β à une valeur de température la plus petite incluse dans le premier groupe de valeurs de température.

5. Le système de calcul de la température de surface du corps humain selon l'une quelconque des revendications 1 à 3, dans lequel l'IA de détection de personne est une IA de détection d'objet.

6. Le système de calcul de la température de surface du corps humain selon l'une quelconque des revendications 1 à 3, dans lequel l'IA de détection de personne est une IA d'estimation de posture.

7. Le système de calcul de la température de surface du corps humain selon la revendication 6, dans lequel la zone de détection de personne est une zone rectangulaire entourée par des lignes qui relient, parmi une pluralité de noeuds inclus dans un modèle d'ossature d'un corps de la personne détectée dans l'image thermique par l'IA d'estimation de posture, des noeuds le plus externe pour les directions haut, bas, gauche et droite de l'image thermique.

8. Le système de calcul de la température de surface du corps humain selon la revendication 6, dans lequel
en plus d'un premier procédé d'extraction du second groupe de valeurs de température sur la base de la règle, le second calculateur est capable d'effectuer l'extraction du second groupe de valeurs de température par un second procédé, et
dans le second procédé, le second calculateur effectue l'extraction, en tant que second groupe de valeurs de température, d'un groupe de valeurs de température, à partir du premier groupe de valeurs de température, le groupe de valeurs de température étant inclus dans une zone définie par des lignes reliant différents noeuds dans un modèle d'ossature d'un corps de la personne détectée dans l'image thermique par l'IA d'estimation de posture.

9. Un procédé de calcul de la température de surface du corps humain exécuté par un ordinateur, le procédé de calcul de la température de surface du corps humain comprenant :
la génération d'une image thermique d'un espace cible sur la base de données de distribution de température indiquant une distribution de température de l'espace cible obtenue par un capteur infrarouge ;
l'identification d'une zone de détection de personne à l'aide d'une intelligence artificielle de détection de personne (IA), la zone de détection de personne étant une zone rectangulaire qui comprend une personne visible dans l'image thermique ;
l'extraction d'un premier groupe de valeurs de température correspondant à la zone de détection de personne à partir des données de distribution de température, et le calcul, sur la base du premier groupe de valeurs de température extrait, d'une température d'une zone d'arrière-plan et d'une température moyenne de la zone de détection de personne, la zone d'arrière-plan étant une zone qui est incluse dans la zone de détection de personne et dans laquelle un arrière-plan à l'exclusion de la personne est visible ; et
l'extraction d'un second groupe de valeurs de température à partir du premier groupe de valeurs de température sur la base d'une règle déterminée par une relation de grandeur entre la température de la zone d'arrière-plan calculée et la température moyenne de la zone de détection de personne calculée, et le calcul d'une température de surface de la personne sur la base du second groupe de valeurs de température extrait.

10. Un programme destiné à amener l'ordinateur à exécuter le procédé de calcul de la température de surface du corps humain selon la revendication 9.
